# EUROPEAN PATENT SPECIFICATION

(11) **EP 3 165 905 B1**
(45) Date of publication and mention of the grant of the patent: **02.10.2019**
(21) Application number: 15815028.4
(22) Date of filing: 11.06.2015
(51) Int. Cl.: G01N 21/61, G01N 21/3504, G01N 33/00

(54) **GAS CONCENTRATION MEASUREMENT DEVICE**
GASKONZENTRATIONSMESSVORRICHTUNG
DISPOSITIF DE MESURE DE CONCENTRATION DE GAZ

(30) Priority: 03.07.2014 JP 2014137787
(43) Date of publication of application: 10.05.2017
(73) Proprietor: Murata Manufacturing Co., Ltd., Nagaokakyo-shi, Kyoto 617-8555 (JP)
(72) Inventor: YASUDA, Masaaki, Nagaokakyo-shi Kyoto 617-8555 (JP); IKEDA, Yoshinori, Nagaokakyo-shi Kyoto 617-8555 (JP)
(74) Representative: Reeve, Nicholas Edward
(86) International application number: PCT/JP2015/066873
(87) International publication number: WO 2016/002467

(56) References cited:
- JP-A- 2004 117 259
- JP-A- 2007 501 404
- JP-A- 2007 501 404
- JP-A- 2010 223 610
- JP-A- 2012 202 918
- KR-B1- 100 791 961
- US-A- 4 084 909

## Description

### Technical Field

The present invention relates to an infrared-light-absorption gas concentration measurement device.

### Background of the Invention

Various types of gas concentration measurement devices for analyzing various types of exhaust, gas contained in the atmosphere, or gas contained in the air in a building have been developed. In particular, infrared-light-absorption gas concentration measurement devices are used to analyze sample gas by utilizing the fact that the sample gas absorbs infrared light in a specific wavelength range.

International Publication No. WO01/27596, for example, discloses an infrared-light-absorption gas concentration measurement device. In the gas concentration measurement device disclosed in WO01/27596, an anti-reflection film is applied to inner walls of an analysis chamber (sample cell) that defines a flow channel for the sample gas. Accordingly, the risk that the infrared light will be incident on a band pass filter, which is provided on a surface of a detector, at an angle greater than a predetermined angle is reduced.

The anti-reflection film disclosed in WO01/27596 needs to be made of a material having a reflectance close to zero. Thus, the material of the anti-reflection film is limited. If an inexpensive material having a reflectance that is not close to zero is used, the infrared light is reflected by the anti-reflection film and is incident on the band pass filter at an angle greater than the predetermined angle. In such a case, the transmission band of the band pass filter is shifted and the measurement accuracy decreases.

KR100791961 B1 discloses an existing undifferentiated acid infrared gas analyzer (Non-dispersive Infrared Gas Analyzer: NDIR) device that includes a light source that emits infrared rays in only one direction; a tapered waveguide; a gas medium; and a sensor.

JP2010223610A discloses a sensor using an optical waveguide core. This sensor includes a light source, an optical fiber, a probe, an optical fiber, and a detector. The probe includes an optical waveguide core having a down taper, and a clad for adsorbing a specific chemical substance and swelling. The quantity of light of an optical component reflected on an interface between the optical waveguide core and the clad 322 adsorbing the specific chemical substance and swelling increases when the refractive index of the clad for adsorbing the specific chemical substance and swelling decreases. There is an extremely small amount of component transmitted to the optical fiber without being reflected on the interface between the optical waveguide core and the clad, so that the sensing sensitivity of the sensor improves comparing with that of a sensor using an optical waveguide core having no taper.

JP2007501404A discloses a radiation sensor, for example, for non-contact temperature measurement, or infra-red spectroscopy, with a detector element, comprising an absorber element, which absorbs radiation and thus heats up, and a support body with a support body surface, for housing the absorber element. The support body surface has a recess and the absorber element is arranged on the support body surface and over the recess such that at least a section of the absorber element is not in contact with the support body. According to the invention, a radiation sensor of the above type may be provided, which generates an amplified signal on the smallest possible chip surface, which permits a small measurement point and which may be produced by conventional standardised technology, whereby the recess has a size corresponding to at least 45 % of the support body surface.

Therefore, there is a need for a gas concentration measurement device having a new structure that can be substituted for the anti-reflection film made of a specified material and that can reduce the risk that the infrared light will be incident on the band pass filter at an angle greater than the predetermined angle.

The present invention has been made in light of the above-described problem, and we have appreciated that it would be desirable to provide a gas concentration measurement device capable of increasing the measurement accuracy.

### SUMMARY OF THE INVENTION

A gas concentration measurement device according to the present invention is defined by the appended claims and includes a light source that emits infrared light; a band pass filter; a detector that detects the infrared light from the light source through a band pass filter; and a waveguide member including a wave-guiding portion having a tubular inner peripheral surface, an entrance portion that is formed at one side of the wave-guiding portion and through which the infrared light from the light source is introduced, and an exit portion that is formed at the other side of the wave-guiding portion and guides the infrared light that has passed through the wave-guiding portion toward the detector. A portion or entirety of the inner peripheral surface of the wave-guiding portion includes a tapered region having a cross section that decreases along a longitudinal axis direction from the entrance portion to the exit portion. The tapered region of the inner peripheral surface of the wave-guiding portion includes a first curved portion. The first curved portion has a curvature in the longitudinal axis direction from the entrance portion to the exit portion and a cross section that decreases along the longitudinal axis direction from the entrance portion to the exit portion. The waveguide member reflects the infrared light that has entered the wave-guiding portion through the entrance portion in the tapered region, so that energy of the infrared light that is obliquely incident on the band pass filter is reduced.

In the gas concentration measurement device according to the present invention, preferably, the tapered region of the inner peripheral surface of the wave-guiding portion includes a truncated conical or pyramidal portion having a perimeter that decreases along the longitudinal axis direction from the entrance portion to the exit portion.

In the gas concentration measurement device according to the present invention, preferably, an opening area of the entrance portion is greater than an opening area of the exit portion.

In the gas concentration measurement device according to the present invention, preferably, a length of the first curved portion in the longitudinal axis direction of the wave-guiding portion is greater than or equal to half a length of the wave-guiding portion in the longitudinal axis direction of the wave-guiding portion.

In the gas concentration measurement device according to the present invention, preferably, the inner peripheral surface of the wave-guiding portion includes a second curved portion having a curvature in the longitudinal axis direction and a cross section that increases in the longitudinal axis direction from the entrance portion to the exit portion up to a position of maximum circumference; wherein the cross section of the first curved portion decreases in the longitudinal axis direction from the entrance portion to the exit portion from the position of maximum circumference.

In the gas concentration measurement device according to the present invention, preferably, the first curved portion and the second curved portion form a portion of a spherical surface located between the entrance portion and the exit portion of the waveguide member.

In the gas concentration measurement device according to the present invention, preferably, the waveguide member is made of a resin material.

According to the present invention, a gas concentration measurement device capable of increasing the measurement accuracy can be provided.

### BRIEF DESCRIPTION OF THE DRAWINGS

Fig. 1 is a schematic sectional view of a gas concentration measurement device according to a first example, not covered by the claimed invention;
Fig. 2 is a schematic sectional view of a gas concentration measurement device according to a first embodiment.
Fig. 3 is a schematic sectional view of a gas concentration measurement device according to a second embodiment.
Fig. 4 is a schematic sectional view of a gas concentration measurement device according to a second example, not covered by the claimed invention;
Fig. 5 is a schematic sectional view of a gas concentration measurement device according to a third example, not covered by the claimed invention;

### DETAILED DESCRIPTION OF EXAMPLE EMBODIMENTS

Embodiments of the present invention and examples will be described in detail with reference to the drawings. In the embodiments and examples described below, components that are the same or similar are denoted by the same reference numerals in the drawings, and descriptions thereof will not be repeated.

### First Embodiment

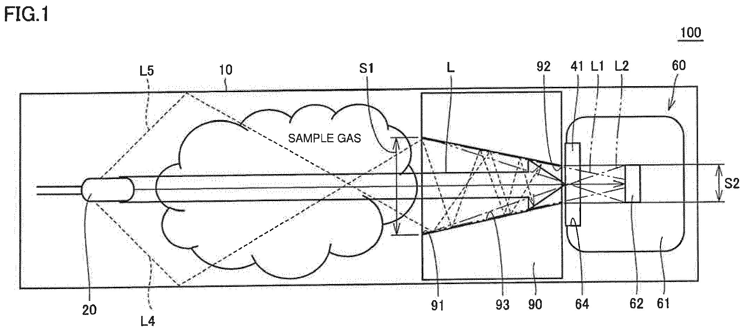

Fig. 1 is a schematic sectional view of a gas concentration measurement device according to the first example. The gas concentration measurement device according to the present example will be described with reference to Fig. 1.

As illustrated in Fig. 1, a gas concentration measurement device 100 according to the example includes a sample cell 10, a light source 20, a band pass filter 41, a detector 60, and a waveguide member 90. The gas concentration measurement device 100 measures a gas concentration in accordance with the absorbance of sample gas that flows through a space between the light source 20, which emits infrared light, and the detector 60, which includes a light-receiving portion 62 that receives the infrared light.

The sample cell 10 includes a sample-gas flow space and allows the sample gas to flow therethrough. For example, a sample-gas introduction hole (not shown) is connected to one end of the sample cell 10 (end close to the light source 20), and a sample-gas discharge hole (not shown) is connected to the other end of the sample cell 10 (end close to the detector 60). The sample gas introduced into the sample cell 10 through the sample-gas introduction hole is discharged through the sample-gas discharge hole.

The sample cell 10 contains the light source 20, the waveguide member 90, the band pass filter 41, and the detector 60. The light source 20, the waveguide member 90, the band pass filter 41, and the detector 60 are arranged, for example, in that order from one end of the sample cell 10 (from the left side in the figure).

The light source 20 emits infrared light. The light source 20 may be, for example, a filament lamp or an LED lamp that emits wide-band infrared light including desired infrared light. Part of the infrared light emitted from the light source 20 is absorbed depending on infrared light absorption wavelength characteristics of the sample gas. The sample gas is, for example, carbon dioxide, and the absorption band thereof is about 4.3 µm.

The waveguide member 90 includes a wave-guiding portion 93 having a tubular inner peripheral surface; an entrance portion 91 that is formed at one end of the wave-guiding portion 93 and through which the infrared light from the light source 20 is introduced; and an exit portion 92 that is formed at the other end of the wave-guiding portion 93 and guides the infrared light that has passed through the wave-guiding portion 93 toward the detector 60. The waveguide member 90 guides the infrared light toward the detector 60 after part of the infrared light is absorbed by the sample gas.

The inner peripheral surface of the wave-guiding portion 93 includes a tapered region having a cross-sectional area that decreases from the entrance portion 91 toward the exit portion 92. The tapered region has a truncated conical or pyramidal shape whose circumference decreases from the entrance portion 91 toward the exit portion 92. The truncated conical or pyramidal shape includes a truncated conical shape and a truncated polygonal pyramidal shape.

The waveguide member 90 may be made of a resin material, such as acrylonitrile butadiene styrene copolymer synthetic resin (ABS resin) or polycarbonate resin (PC resin). In particular, the waveguide member 90 is preferably made of a resin material having a reflectance of 20% or less in the infrared range.

The band pass filter 41 is provided at an end of the detector 60 that is adjacent to the waveguide member 90. The band pass filter 41 is securely fitted in a recess 64 formed in a surface of the detector 60 that faces the waveguide member 90.

The band pass filter 41 is configured to transmit the infrared light in an absorption band of the sample gas to be detected. Thus, only the infrared light having a desired wavelength band reaches the detector 60.

The detector 60 may be an infrared light detector, such as a thermopile or a bolometer. The detector 60 includes a main portion 61, a light-receiving portion 62, and the recess 64. The light-receiving portion 62 is embedded in the main portion 61. The light-receiving portion 62 receives the infrared light guided out of the exit portion 92 of the waveguide member 90 through the band pass filter 41.

The detector 60 is electrically connected to a signal processing circuit board (not shown). The detector 60 outputs an output signal to the signal processing circuit board based on the infrared light received by the light-receiving portion 62. The signal processing circuit board calculates the concentration of the sample gas based on the output signal.

The infrared light incident on the band pass filter 41 will now be described. In general, the transmission band of the band pass filter 41 shifts toward the short-wavelength side as the incident angle increases. The measurement accuracy decreases when the transmission band varies. Therefore, when the measurement is performed, the incident angle of the infrared light incident on the band pass filter 41 is preferably small.

In the present embodiment, the waveguide member 90 is provided to suppress the influence on the measurement accuracy of the infrared light that is incident on the band pass filter 41 located at the transmitting position at a large incident angle.

The infrared light that linearly travels through the region that is the logical sum of the region surrounded by the outermost rays of infrared light L1 and the region surrounded by the outermost rays of infrared light L2 (for example, arrow L in Fig. 1) mainly reaches the light-receiving portion 62 through the band pass filter 41. The infrared light L1 is light having a truncated conical or pyramidal shape that linearly travels along the peripheral surface of the wave-guiding portion 93 toward the detector 60. The infrared light L2 is light having a shape including two truncated cones obtained by rotating, for example, a ray of light that linearly travels from the bottom end of the entrance portion 91 of the waveguide member 90 in Fig. 1 to the top end of the light-receiving portion 62 in Fig. 1, one turn along the opening shape of the entrance portion 91.

The infrared light that linearly travels through the region that is the logical sum of the region surrounded by the outermost rays of the infrared light L1 and the region surrounded by the outermost rays of the infrared light L2 passes through the band pass filter 41 at a relatively small angle.

Infrared light L4 and infrared light L5 enter the wave-guiding portion 93 through the entrance portion 91 at a relatively large angle with respect to the axial direction of the wave-guiding portion 93. The infrared light L4 and the infrared light L5 travel toward the detector 60 while being reflected by the inner peripheral surface of the wave-guiding portion 93 a plurality of times.

If it is assumed that the reflectance of the wave-guiding portion 93 is 100%, the infrared light L4 and the infrared light L5 may be incident on the band pass filter 41 at a large incident angle.

In the present example, the waveguide member 90 is made of a resin material having a reflectance of 20% or less. Therefore, the infrared light L4 and the infrared light L5 are absorbed and attenuated by being repeatedly reflected in the wave-guiding portion 93. For example, in the case where the reflectance of the material is 10%, the attenuation effect obtained when the infrared light is reflected five times is similar to that obtained when the infrared light is reflected once by a component having a reflectance of 0.001%. The number of times the infrared light L4 and the infrared light L5 are reflected can be increased by setting an opening area S1 of the entrance portion 91 of the waveguide member 90 greater than an opening area S2 of the exit portion 92 and forming the wave-guiding portion 93 so that the wave-guiding portion 93 includes a tapered region.

Thus, the waveguide member 90 repeatedly reflects the infrared light that has entered the wave-guiding portion 93 through the entrance portion 91 in the tapered region, thereby reducing the energy of the infrared light that is obliquely incident on the band pass filter 41. Accordingly, the measurement accuracy of the gas concentration measurement device can be increased.

As described above, in the gas concentration measurement device 100 according to the present embodiment, since the waveguide member 90 is provided, the energy of the obliquely incident infrared light can be reduced. Therefore, the measurement accuracy of the gas concentration measurement device 100 can be increased.

Although the sample gas is carbon dioxide in the present embodiment, the sample gas is not limited to this, and may instead be, for example, carbon monoxide, CH₄, or NOₓ.

### First Embodiment

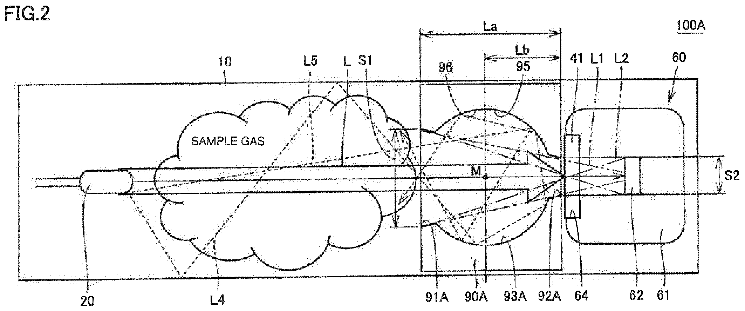

Fig. 2 is a schematic sectional view of a gas concentration measurement device according to the present embodiment. A gas concentration measurement device 100A according to the present embodiment will be described with reference to Fig. 2.

As illustrated in Fig. 2, the gas concentration measurement device 100A according to the present embodiment includes a waveguide member 90A including a wave-guiding portion 93A having a shape that differs from that in the gas concentration measurement device 100 according to the first example. Other structures are substantially the same as those in the first example.

Also in the waveguide member 90A, an opening area S1 of an entrance portion 91A is greater than an opening area S2 of an exit portion 92A. The internal shape of the wave-guiding portion 93A is partially spherical. A portion of the inner peripheral surface of the wave-guiding portion 93A that forms a portion of a spherical surface is located between the entrance portion 91A and the exit portion 92A.

The portion that forms a portion of a spherical surface includes a first spherical surface portion 95, which extends toward the exit portion 92A from a boundary at the center M of the sphere, and a second spherical surface portion 96, which extends toward the entrance portion 91A from the boundary at the center M. The region from the first spherical surface portion 95 to the exit portion 92A corresponds to a tapered region having a cross-sectional area that decreases along the direction from the entrance portion 91A to the exit portion 92A, and also corresponds to a first curved portion having a circumference that decreases along the direction from the entrance portion 91A to the exit portion 92A. The second spherical surface portion 96 corresponds to a second curved portion having a circumference that decreases along the direction from the exit portion 92A to the entrance portion 91 A.

Also when the wave-guiding portion 93A has the above-described shape, the infrared light that linearly travels through the region that is the logical sum of the region surrounded by the outermost rays of infrared light L1 and the region surrounded by the outermost rays of infrared light L2 (for example, arrow L in Fig. 2) mainly reaches the light-receiving portion 62. The infrared light L1 is light having a truncated conical or pyramidal shape that linearly travels along the peripheral surfaces of the entrance portion 91A and the exit portion 92A toward the detector 60. The infrared light L2 is similar to that in the first example.

Infrared light L4 and infrared light L5 that have entered the wave-guiding portion 93A through the entrance portion 91A at a relatively large angle with respect to the axial direction of the wave-guiding portion 93A are reflected by the first spherical surface portion 95 and the second spherical surface portion 96 a plurality of times and are emitted from the entrance portion 91A.

The infrared light L4 and the infrared light L5 emitted from the entrance portion 91A are not incident on the band pass filter 41. To increase the number of times the light is reflected by the wave-guiding portion 93A, the distance Lb from the first spherical surface portion 95 to the exit portion 92A in the axial direction of the wave-guiding portion 93A is preferably greater than or equal to half the length La of the waveguide member 90.

Thus, in the present embodiment, the energy of the infrared light that is obliquely incident on the band pass filter 41 can be reduced, and part of the obliquely incident infrared light can be emitted from the entrance portion 91A. Accordingly, the measurement accuracy of the gas concentration measurement device 100A can be further increased.

### Second Embodiment

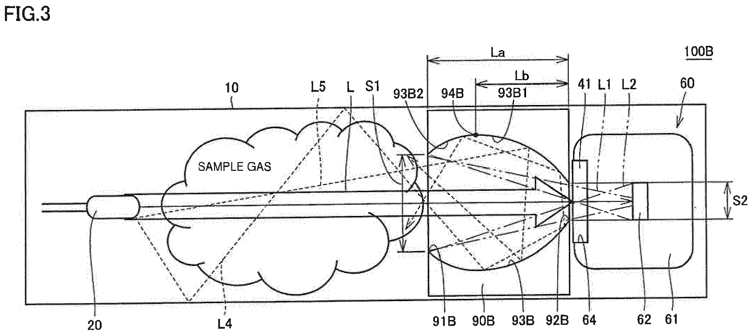

Fig. 3 is a schematic sectional view of a gas concentration measurement device according to the present embodiment. A gas concentration measurement device 100B according to the present embodiment will be described with reference to Fig. 3.

As illustrated in Fig. 3, the gas concentration measurement device 100B according to the present embodiment includes a waveguide member 90B including a wave-guiding portion 93B having a shape that differs from that in the gas concentration measurement device 100 according to the first example. Other structures are substantially the same as those in the first example.

Also in the waveguide member 90B, an opening area S1 of an entrance portion 91B is greater than an opening area S2 of an exit portion 92B. The internal shape of the wave-guiding portion 93B includes a first curved portion 93B1 and a second curved portion 93B2. The wave-guiding portion 93B includes a maximum circumference portion 94B. The maximum circumference portion 94B is located between the entrance portion 91B and the exit portion 92B.

The first curved portion 93B1 defines the internal shape of the wave-guiding portion 93B in a region from the maximum circumference portion 94B to the exit portion 92B. The first curved portion 93B1 has a circumference that decreases along the direction from the entrance portion 91B to the exit portion 92B.

The second curved portion 93B2 defines the internal shape of the wave-guiding portion 93B in a region from the maximum circumference portion 94B to the entrance portion 91B. The second curved portion 93B2 has a circumference that decreases along the direction from the exit portion 92B to the entrance portion 91B.

Also in this case, the infrared light that linearly travels through the region that is the logical sum of the region surrounded by the outermost rays of infrared light L1 and the region surrounded by the outermost rays of infrared light L2 mainly reaches the light-receiving portion 62. The infrared light L1 is light having a truncated conical or pyramidal shape that linearly travels along the peripheral edges of the entrance portion 91B and the exit portion 92B toward the detector 60. The infrared light L2 is similar to that in the first example.

The length Lb of the first curved portion 93B1 in the axial direction of the waveguide member 90B is preferably greater than or equal to half the length La of the waveguide member 90B in the axial direction. When this length relationship is satisfied, infrared light L4 and infrared light L5, which enter through the entrance portion 91B at a relatively large angle with respect to the axial direction of the wave-guiding portion 93B, are reflected a plurality of times in the first curved portion 93B1 and the second curved portion 93B2 and emitted from the entrance portion 91B.

Accordingly, also in the present embodiment, the energy of the infrared light that is obliquely incident on the band pass filter 41 can be reliably reduced. As a result, the measurement accuracy of the gas concentration measurement device 100B can be increased.

### Second Example

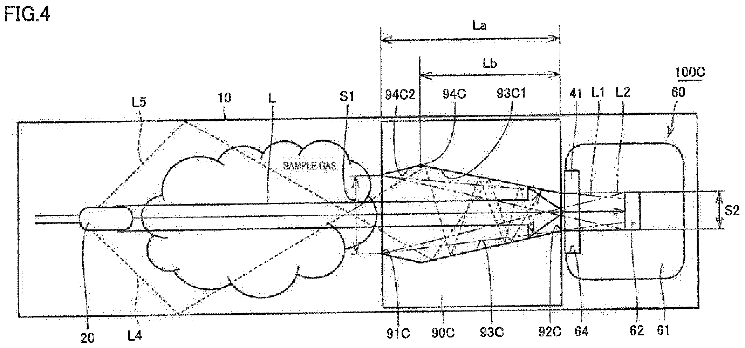

Fig. 4 is a schematic sectional view of a gas concentration measurement device according to the present example. A gas concentration measurement device 100C according to the present example will be described with reference to Fig. 4.

As illustrated in Fig. 4, the gas concentration measurement device 100C according to the present example includes a waveguide member 90C including a wave-guiding portion 93C having a shape that differs from that in the gas concentration measurement device 100 according to the first example. Other structures are substantially the same as those in the first example.

Also in the waveguide member 90C, an opening area S1 of an entrance portion 91C is greater than an opening area S2 of an exit portion 92C. The internal shape of the wave-guiding portion 93C includes a first truncated conical or pyramidal portion 93C1 and a second truncated conical or pyramidal portion 93C2. The wave-guiding portion 93C includes a maximum circumference portion 94C. The maximum circumference portion 94C is located between the entrance portion 91C and the exit portion 92C.

The first truncated conical or pyramidal portion 93C1 defines the internal shape of the wave-guiding portion 93C in a region from the maximum circumference portion 94C to the exit portion 92C. The first truncated conical or pyramidal portion 93C1 has a circumference that decreases along the direction from the entrance portion 91C to the exit portion 92C.

The second truncated conical or pyramidal portion 93C2 defines the internal shape of the wave-guiding portion 93C in a region from the maximum circumference portion 94C to the entrance portion 91C. The second truncated conical or pyramidal portion 93C2 has a circumference that decreases along the direction from the exit portion 92C to the entrance portion 91C.

Also in this case, the infrared light that linearly travels through the region that is the logical sum of the region surrounded by the outermost rays of infrared light L1 and the region surrounded by the outermost rays of infrared light L2 mainly reaches the light-receiving portion 62. The infrared light L1 is light having a truncated conical or pyramidal shape that linearly travels along the peripheral edges of the entrance portion 91C and the exit portion 92C toward the detector 60. The infrared light L2 is similar to that in the first example.

The length Lb of the first truncated conical or pyramidal portion 93C1 in the axial direction of the waveguide member 90C is preferably greater than or equal to half the length La of the waveguide member 90C in the axial direction. When this length relationship is satisfied, infrared light L4, for example, which enters through the entrance portion 91C at a relatively large angle with respect to the axial direction of the wave-guiding portion 93C, is reflected a plurality of times in the first truncated conical or pyramidal portion 93C1 and the second truncated conical or pyramidal portion 93C2.

Accordingly, also in the gas concentration measurement device 100C according to the present example, the energy of the infrared light that is obliquely incident on the band pass filter 41 can be reliably reduced. As a result, the measurement accuracy of the gas concentration measurement device can be increased.

### Third Example

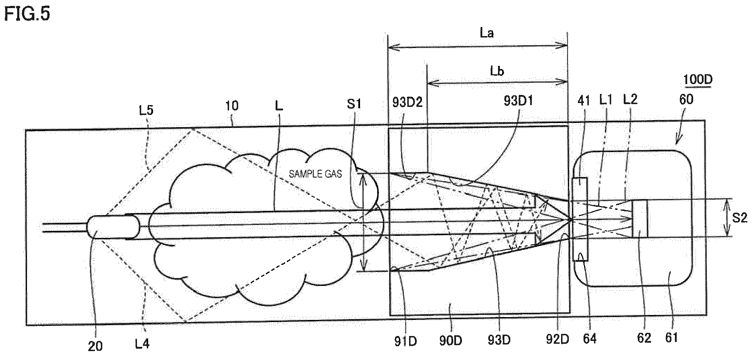

Fig. 5 is a schematic sectional view of a gas concentration measurement device according to the present example. A gas concentration measurement device 100D according to the present example will be described with reference to Fig. 5.

As illustrated in Fig. 5, the gas concentration measurement device 100D according to the present example includes a waveguide member 90D including a wave-guiding portion 93D having a shape that differs from that in the gas concentration measurement device 100C according to the second example.

Also in the waveguide member 90D, an opening area S1 of an entrance portion 91D is greater than an opening area S2 of an exit portion 92D. The internal shape of the wave-guiding portion 93D includes a truncated conical or pyramidal portion 93D1 and a columnar portion 93D2. The shape of the truncated conical or pyramidal portion 93D1 is substantially the same as that of the first truncated conical or pyramidal portion 93C1 according to the second example. The relationship between the length of the truncated conical or pyramidal portion 93D1 and the length of the waveguide member 90D is also similar to the relationship between the length of the first truncated conical or pyramidal portion 93C1 and the length of the waveguide member 90C according to the second example.

The columnar portion 93D2 is configured so that the circumference thereof is constant over a region from the entrance portion 91D to the upstream end of the truncated conical or pyramidal portion 93D1. The circumference of the columnar portion 93D2 is equal to the maximum circumference.

Also in this case, infrared light L4 and infrared light L5, which enter through the entrance portion 91D at a relatively large angle with respect to the axial direction of the wave-guiding portion 93D, is reflected a plurality of times in the truncated conical or pyramidal portion 93D1 and the columnar portion 93D2.

Accordingly, also in the gas concentration measurement device 100D according to the present example, the energy of the infrared light that is obliquely incident on the band pass filter 41 can be reliably reduced. As a result, the measurement accuracy of the gas concentration measurement device can be increased.

In the present example, the columnar portion is arranged adjacent to the entrance portion 91D, and the truncated conical or pyramidal portion is arranged adjacent to the exit portion 92D. However, the arrangement is not limited to this, and the columnar portion may be arranged adjacent to the exit portion 92D while the truncated conical or pyramidal portion is arranged adjacent to the entrance portion 91D. In this case, the circumference of the columnar portion is set to the minimum circumference.

In the above-described second embodiment, a portion that connects the entrance portion 91A to the upstream end of the second spherical surface portion 96 (first connecting portion) and a portion that connects the downstream end of the first spherical surface portion 95 to the exit portion 92A (second connecting portion) have circumferences that decrease along the direction from the entrance portion 91A to the exit portion 92A. However, the first and second connecting portions are not limited to this, and at least one of the first and second connecting portions may have a columnar shape with a constant circumference.

In the above-described third embodiment, the second curved portion 93B2 is provided so as to extend from the entrance portion 91B to the maximum circumference portion 94B. However, the second curved portion 93B2 may be replaced by a columnar portion having a circumference that is constant over a region from the entrance portion 91B to the maximum circumference portion 94B.

Although embodiments of the present invention and examples have been described, the embodiments disclosed herein are illustrative and not restrictive in all points. The scope of the present invention is to be defined by the scope of the claims, and includes equivalents to the scope of the claims and all changes within the scope of the claims.

### Reference Signs List

10 sample cell, 20 light source, 41 band pass filter, 60 detector, 61 main portion, 62 light-receiving portion, 64 recess, 90, 90A, 90B, 90C, and 90D waveguide member, 91, 91A, 91B, 91C, and 91D entrance portion, 92, 92A, 92B, 92C, and 92D exit portion, 93, 93A, 93B, 93C, and 93D wave-guiding portion, 93B1 first curved portion, 93B2 second curved portion, 93C1 first truncated conical or pyramidal portion, 93C2 second truncated conical or pyramidal portion, 93D1 truncated conical or pyramidal portion, 93D2 columnar portion, 94B and 94C maximum circumference portion, 95 first spherical surface portion, 96 second spherical surface portion, 100, 100A, 100B, 100C, and 100D gas concentration measurement device.

## Claims

1. A gas concentration measurement device (100) comprising:
a light source (20) that emits infrared light;
a band pass filter (41);
a detector (60) that detects the infrared light from the light source through the band pass filter; and
a waveguide member (90) including a wave-guiding portion (93) having a tubular inner peripheral surface, an entrance portion (91) that is formed at one side of the wave-guiding portion and through which the infrared light from the light source is introduced, and an exit portion (92) that is formed at the other side of the wave-guiding portion and configured to guide the infrared light that has passed through the wave-guiding portion toward the band pass filter and subsequently to the detector,
wherein a portion or entirety of the inner peripheral surface of the wave-guiding portion includes a tapered region having a cross section that decreases along a longitudinal axis direction from the entrance portion to the exit portion,
wherein the tapered region of the inner peripheral surface of the wave-guiding portion includes a first curved portion (93A, 93B1);
the first curved portion having a curvature in the longitudinal axis direction from the entrance portion to the exit portion and a cross section that decreases along the longitudinal axis direction from the entrance portion to the exit portion; and
wherein the waveguide member is configured to reflect the infrared light that has entered the wave-guiding portion through the entrance portion in the tapered region, so that energy of the infrared light that is obliquely incident on the band pass filter is reduced.

2. The gas concentration measurement device (100) according to claim 1, wherein the tapered region of the inner peripheral surface of the wave-guiding portion (93) includes a truncated conical or pyramidal portion having a perimeter that decreases along the longitudinal axis direction from the entrance portion (91) to the exit portion (92).

3. The gas concentration measurement device (100) according to claim 1 or 2, wherein an opening area of the entrance portion (91) is greater than an opening area of the exit portion (92).

4. The gas concentration measurement device (100) according to any preceding claim, wherein the length of the first curved portion (93A, 93B1) in the longitudinal direction of the wave-guiding portion (93) is greater than or equal to half a length of the wave-guiding portion in the longitudinal axis direction of the wave-guiding portion.

5. The gas concentration measurement device (100) according to any preceding claim, wherein the inner peripheral surface of the wave-guiding portion (93) includes a second curved portion (93B2, 96) having a curvature in the longitudinal axis direction and a cross section that increases in the longitudinal axis direction from the entrance portion to the exit portion up to a position of maximum circumference (94);
wherein the cross section of the first curved portion (93A, 93B1) decreases in the longitudinal axis direction from the entrance portion to the exit portion from the position of maximum circumference.

6. The gas concentration measurement device (100) of claim 5, wherein the first curved portion (93A, 93B1) and the second curved portion (93B2, 96) form a portion of a spherical surface (95, 96) located between the entrance portion (91) and the exit portion (92) of the waveguide member.

7. The gas concentration measurement device (100) according to any one of claims 1 to 6, wherein the waveguide member (90) is made of a resin material.

## Patentansprüche

1. Gaskonzentrationsmessvorrichtung (100), die Folgendes umfasst:
eine Lichtquelle (20), die Infrarotlicht ausstrahlt;
ein Bandpassfilter (41);
einen Detektor (60), der das Infrarotlicht von der Lichtquelle durch das Bandpassfilter erkennt; und
ein Wellenleitelement (90) einschließlich eines Wellenleitabschnitts (93) mit einer rohrförmigen inneren Umfangsfläche, einem Eintrittsabschnitt (91), der auf einer Seite des Wellenleitabschnitts ausgebildet ist und durch den das Infrarotlicht von der Lichtquelle eingeleitet wird, und einem Austrittsabschnitt (92), der auf der anderen Seite des Wellenleitabschnitts ausgebildet und zum Leiten des Infrarotlichts, das durch den Wellenleitabschnitt passiert ist, zum Bandpassfilter und nachfolgend zum Detektor konfiguriert ist,
wobei ein Abschnitt oder die Gesamtheit der inneren Umfangsfläche des Wellenleitabschnitts eine konische Region mit einem Querschnitt aufweist, der entlang einer Längsachsenrichtung vom Eintrittsabschnitt zum Austrittsabschnitt abnimmt,
wobei die konische Region der inneren Umfangsfläche des Wellenleitabschnitts einen ersten gekrümmten Abschnitt (93A, 93B1) aufweist;
wobei der erste gekrümmte Abschnitt eine Krümmung in der Längsachsenrichtung vom Eintrittsabschnitt zum Austrittsabschnitt und einen Querschnitt aufweist, der entlang der Längsachsenrichtung vom Eintrittsabschnitt zum Austrittsabschnitt abnimmt; und
wobei das Wellenleitelement so konfiguriert ist, dass es das Infrarotlicht reflektiert, das durch den Eintrittsabschnitt in der konischen Region in den Wellenleitabschnitt eingetreten ist, so dass Energie des Infrarotlichts, das schräg auf das Bandpassfilter einfällt, reduziert wird.

2. Gaskonzentrationsmessvorrichtung (100) nach Anspruch 1, wobei die konische Region der inneren Umfangsfläche des Wellenleitabschnitts (93) einen kegelstumpfförmigen oder pyramidenförmigen Abschnitt mit einem Perimeter aufweist, der entlang der Längsachsenrichtung vom Eintrittsabschnitt (91) zum Austrittsabschnitt (92) abnimmt.

3. Gaskonzentrationsmessvorrichtung (100) nach Anspruch 1 oder 2, wobei ein Öffnungsbereich des Eintrittsabschnitts (91) größer ist als ein Öffnungsbereich des Austrittsabschnitts (92).

4. Gaskonzentrationsmessvorrichtung (100) nach einem vorherigen Anspruch, wobei die Länge des ersten gekrümmten Abschnitts (93A, 93B1) in der Längsrichtung des Wellenleitabschnitts (93) genauso groß wie oder größer als eine Hälfte einer Länge des Wellenleitabschnitts in der Längsachsenrichtung des Wellenleitabschnitts ist.

5. Gaskonzentrationsmessvorrichtung (100) nach einem vorherigen Anspruch, wobei die innere Umfangsfläche des Wellenleitabschnitts (93) einen zweiten gekrümmten Abschnitt (93B2, 96) mit einer Krümmung in der Längsachsenrichtung und einem Querschnitt hat, der in der Längsachsenrichtung vom Eintrittsabschnitt zum Austrittsabschnitt bis zu einer Position mit maximalem Umfang (94) zunimmt;
wobei der Querschnitt des ersten gekrümmten Abschnitts (93A, 93B1) in der Längsachsenrichtung vom Eintrittsabschnitt zum Austrittsabschnitt von der Position mit maximalem Umfang abnimmt.

6. Gaskonzentrationsmessvorrichtung (100) nach Anspruch 5, wobei der erste gekrümmte Abschnitt (93A, 93B1) und der zweite gekrümmte Abschnitt (93B2, 96) einen Abschnitt einer sphärischen Oberfläche (95, 96) bilden, der sich zwischen dem Eintrittsabschnitt (91) und dem Austrittsabschnitt (92) des Wellenleitelements befindet.

7. Gaskonzentrationsmessvorrichtung (100) nach einem der Ansprüche 1 bis 6, wobei das Wellenleitelement (90) aus einem Harzmaterial besteht.

## Revendications

1. Dispositif de mesure de concentration de gaz (100) comprenant:
une source de lumière (20) qui émet une lumière infrarouge ;
un filtre passe-bande (41) ;
un détecteur (60) qui détecte la lumière infrarouge provenant de la source de lumière à travers le filtre passe-bande ; et
un élément guide d'onde (90) comportant une partie de guidage d'onde (93) présentant une surface périphérique interne tubulaire, une partie d'entrée (91) qui est formée d'un côté de la partie de guidage d'onde et à travers laquelle la lumière infrarouge provenant de la source de lumière est introduite, et une partie de sortie (92) qui est formée de l'autre côté de la partie de guidage d'onde et est configurée pour guider la lumière infrarouge qui a traversé la partie de guidage d'onde vers le filtre passe-bande puis jusqu'au détecteur,
dans lequel une partie ou la totalité de la surface périphérique interne de la partie de guidage d'onde comporte une région conique présentant une coupe transversale qui diminue le long d'un sens d'axe longitudinal depuis la partie d'entrée jusqu'à la partie de sortie,
dans lequel la région conique de la surface périphérique interne de la partie de guidage d'onde comporte une première partie incurvée (93A, 93B1) ;
la première partie incurvée présentant une courbure dans le sens d'axe longitudinal depuis la partie entrée jusqu'à la partie de sortie et une coupe transversale qui diminue le long du sens d'axe longitudinal depuis la partie d'entrée jusqu'à la partie de sortie ; et
dans lequel l'élément guide d'onde est configuré pour réfléchir la lumière infrarouge qui est entrée dans la partie de guidage d'onde à travers la partie d'entrée dans la région conique, de manière à réduire l'énergie de la lumière infrarouge qui frappe de manière oblique le filtre passe-bande.

2. Dispositif de mesure de concentration de gaz (100) selon la revendication 1, dans lequel la région amincie de la surface périphérique interne de la partie de guidage d'onde (93) comporte une partie tronconique ou pyramidale présentant un périmètre qui diminue le long du sens d'axe longitudinal depuis la partie d'entrée (91) jusqu'à la partie de sortie (92).

3. Dispositif de mesure de concentration de gaz (100) selon la revendication 1 ou 2, dans lequel une zone d'ouverture de la partie d'entrée (91) est plus grande qu'une zone d'ouverture de la partie de sortie (92).

4. Dispositif de mesure de concentration de gaz (100) selon n'importe quelle revendication précédente, dans lequel la longueur de la première partie incurvée (93A, 93B1) dans le sens longitudinal de la partie de guidage d'onde (93) est supérieure ou égale à une demi-longueur de la partie de guidage d'onde dans le sens d'axe longitudinal de la partie de guidage d'onde.

5. Dispositif de mesure de concentration de gaz (100) selon n'importe quelle revendication précédente, dans lequel la surface périphérique interne de la partie de guidage d'onde (93) comporte une seconde partie incurvée (93B2, 96) présentant une courbure dans le sens d'axe longitudinal et une coupe transversale qui augmente dans le sens d'axe longitudinal depuis la partie d'entrée jusqu'à la partie de sortie jusqu'à une position de circonférence maximale (94) ;
dans lequel la coupe transversale de la première partie incurvée (93A, 93B1) diminue dans le sens d'axe longitudinal depuis la partie d'entrée jusqu'à la partie de sortie depuis la position de circonférence maximale.

6. Dispositif de mesure de concentration de gaz (100) selon la revendication 5, dans lequel la première partie incurvée (93A, 93B1) et la seconde partie incurvée (93B2, 96) forment une partie d'une surface sphérique (95, 96) située entre la partie d'entrée (91) et la partie de sortie (92) de l'élément guide d'onde.

7. Dispositif de mesure de concentration de gaz (100) selon l'une quelconque des revendications 1 à 6, dans lequel l'élément guide d'onde (90) est réalisé en un matériau de résine.
